# EUROPÄISCHE PATENTSCHRIFT

(11) **EP 2 903 494 B1**
(45) Veröffentlichungstag und Bekanntmachung des Hinweises auf die Patenterteilung: **09.01.2019**
(21) Anmeldenummer: 13782688.9
(22) Anmeldetag: 22.10.2013
(51) Int. Cl.: A61B 10/04, A61B 1/273, A61B 1/00, A61B 1/018, A61B 10/06

(54) **ENDOSKOPIE- UND BIOPSIE-EINWEGSYSTEM**
DISPOSABLE ENDOSCOPY AND BIOPSY SYSTEM
SYSTÈME D'ENDOSCOPIE ET DE BIOPSIE À USAGE UNIQUE

(30) Priorität: 23.10.2012 DE 102012219342
(43) Veröffentlichungstag der Anmeldung: 12.08.2015
(73) Patentinhaber: INGOSCOPE SYSTEMS GmbH, 13187 Berlin (DE)
(72) Erfinder: HERRMANN, Ingo F., 81479 München (DE)
(74) Vertreter: Manitz Finsterwald Patentanwälte PartmbB
(86) Internationale Anmeldenummer: PCT/EP2013/072072
(87) Internationale Veröffentlichungsnummer: WO 2014/064105

(56) Entgegenhaltungen:
- DE-A1-102007 008 099
- DE-A1-102010 049 568
- US-A- 5 683 413
- US-A1- 2006 149 131
- US-A1- 2008 051 631

## Beschreibung

Die Erfindung betrifft ein Endoskopie- und Biopsie-Einwegsystem, mit einem Einwegschlauch, der zumindest einen Arbeitskanal umgibt und der Endoskopbefestigungsmittel zum Befestigen des Einwegschlauchs an einem Endoskop aufweist, und wenigstens einer Einwegbiopsiezange, die in den Arbeitskanal einführbar oder eingeführt ist, wobei die Einwegbiopsiezange einen Zangenkopf mit wenigstens zwei beweglichen Backen und eine Bowdenzugseele zum Betätigen der Backen aufweist.

Der Einwegschlauch einschließlich des Arbeitskanals kann dabei über die Endoskopbefestigungsmittel seitlich am Endoskop befestigt werden. Die Endoskopbefestigungsmittel umfassen beispielsweise eine Fixiereinrichtung für ein drehfestes und axial festes Fixieren am distalen Ende des Endoskops und/oder eine oder mehrere Führungsschlaufen, die für eine längsverschiebliche Befestigung beabstandet voneinander entlang der Länge des Einwegschlauchs angeordnet sind.

Die Biopsiezange kann zur Entnahme einer Gewebeprobe dienen, beispielsweise aus der Speiseröhre (Ösophagus) oder aus dem Magen des Patienten, insbesondere zum Zwecke einer histologischen Untersuchung. Durch die Verwendung eines Einwegschlauchs mit Arbeitskanal wird die Notwendigkeit eines Endoskops vermieden, das einen integrierten Arbeitskanal besitzt und deshalb unter hygienischen Gesichtspunkten unerwünscht sein kann. Mit Hilfe der Endoskopbefestigungsmittel kann der Einwegschlauch an einem Endoskop befestigt werden. Somit kann als Endoskop ein einfaches Fibroskop oder Videoendoskop verwendet werden, das abgesehen von den erforderlichen Bowdenzügen lediglich einen optischen oder elektronischen Beobachtungskanal (jedoch keinen integrierten Arbeitskanal) umfasst und somit einen vorteilhaft geringen Querschnitt besitzt. Ein derartiges Endoskop mitsamt dem genannten Einwegsystem kann dem Patienten z.B. oral oder nasal eingeführt werden.

Ein Einwegschlauch der genannten Art ist generell aus der WO 2008/101653 A2 und der DE 10 2010 049 568 A1 bekannt, auf deren jeweiligen Offenbarungsgehalt ausdrücklich verwiesen wird. Die US 2006/0149131 A1 offenbart eine Einhülleinrichtung, die an einem proximalen Anschluss des Arbeitskanals eines Endoskops befestigt wird und beim Zurückziehen einer Biopsiezange eine flexible Hülle freigibt, die den Schaft der Biopsiezange umschließt.

Die Bowdenzugseele der Biopsiezange kann als ein Betätigungsdraht ausgebildet sein, vorzugsweise aus Metall. Die Bowdenzugseele ist bei einer herkömmlichen Biopsiezange innerhalb einer Führungshülle längsbeweglich geführt, die mit dem Zangenkopf fest verbunden ist. Am distalen Ende der Biopsiezange kann die Bowdenzugseele an die beweglichen Backen des Zangenkopfes unmittelbar oder mittelbar, beispielsweise über eine Umlenk- oder Übersetzungseinrichtung, gekoppelt sein. Bei geschlossenen Backen kann der Außendurchmesser des Zangenkopfes beispielsweise höchstens so groß wie der Innendurchmesser des Arbeitskanals sein. Dadurch wird ein Ein- und/oder Ausführen der Bowdenzugseele in den Arbeitskanal ermöglicht.

Das zugeordnete Endoskop kann - wie bereits erwähnt - einen Beobachtungskanal, insbesondere einen elektronischen Videokanal oder einen optischen Kanal, mit einer Empfangsoptik am distalen Ende umfassen. Ferner kann das zugeordnete Endoskop eine Bowdenzug-Steuereinrichtung zum Krümmen des Endoskops in zwei gewünschte Richtungen umfassen. Dadurch können entsprechende Beobachtungen, Probenentnahmen und/oder ein Saugen oder Spülen in diesen Richtungen erfolgen. Insbesondere kann ein so genanntes Invertieren der Endoskopspitze erfolgen (auch als Inversion bezeichnet), d.h. eine Krümmung um etwa 200° oder nahezu 200°.

Hierbei ist insbesondere wichtig, dass der Einwegschlauch mit dem Arbeitskanal der Krümmung der Endoskopspitze folgen kann, obwohl sich für den Einwegschlauch und die Endoskopspitze unterschiedliche Krümmungsradien ergeben. Dies wird durch die Fixierung am distalen Ende des Endoskops einerseits und die verschiebliche Befestigung entlang der Längserstreckung des Endoskops andererseits ermöglicht.

Problematisch gestaltet sich hierbei jedoch die Steifigkeit einer handelsüblichen Biopsiezange, insbesondere an deren distalem Ende. Dabei auftretende Rückstellkräfte können beispielsweise zu groß sein, um mittels der Bowdenzug-Steuereinrichtung des Endoskops die gewünschten Krümmungsradien des Endoskops einschließlich des Einwegschlauchs und der darin befindlichen Biopsiezange einstellen zu können. Ein weiteres Problem bei der Verwendung einer herkömmlichen Biopsiezange besteht generell unter hygienischen Gesichtspunkten in der schwierigen Reinigung nach der Behandlung.

Es ist eine Aufgabe der Erfindung, die freie Beweglichkeit des Endoskops bei Verwendung eines Einwegschlauchs und einer Biopsiezange noch weiter zu verbessern.

Die Lösung dieser Aufgabe erfolgt durch ein Endoskopie- und Biopsie-Einwegsystem mit den Merkmalen des Anspruchs 1 sowie durch ein Verfahren mit den Merkmalen des Anspruchs 11.

Das erfindungsgemäße Einwegsystem umfasst eine Befestigungseinrichtung zum temporären oder permanenten Befestigen des Zangenkopfes am distalen Ende des Einwegschlauchs in einer Arbeitsposition, in der zumindest die Backen des Zangenkopfes über das distale Ende des Einwegschlauchs hervorragen. Auch ein Teil des Zangenkopfes kann in der Arbeitsposition über das distale Ende des Einwegschlauchs überstehen. Mit anderen Worten ist die Befestigungseinrichtung als Fixpunkt für eine relative Bewegung zwischen der Bowdenzugseele und dem Einwegschlauch und zum Unterbinden einer Bewegung des Zangenkopfes relativ zu dem Einwegschlauch ausgebildet. Somit ist eine Einwegbiopsiezange in den Arbeitskanal des Einwegschlauchs integriert, insbesondere ohne dass eine zusätzliche Führung zwingend erforderlich ist, wie nachstehend noch näher erläutert wird.

Auf diese Weise ergibt sich ein verbessertes Krümmungsverhalten, da zum Führen der Bowdenzugseele der Biopsiezange keine herkömmliche Führungshülle zwingend benötigt wird. Herkömmliche Führungshüllen sind unflexibel und steif, da sie typischerweise aus Metall gefertigt sind, wodurch insbesondere das erläuterte Invertieren nicht oder nur sehr schwer möglich ist. Bei dem erfindungsgemäßen Einwegsystem kann die Führungshülle teilweise oder vollständig durch den Arbeitskanal des Einwegschlauchs ersetzt sein, oder es wird lediglich eine dünne und/oder weiche Führungshülle verwendet, deren Funktion bei Benutzung der Einwegbiopsiezange durch den umgebenden Einwegschlauch unterstützt wird. Der Einwegschlauch kann insbesondere ein Kunststoffmaterial umfassen und somit weich sein, während eine ausreichende Knickfestigkeit oder eine Knickcharakteristik erreicht wird, die eine Beweglichkeit der Bowdenzugseele gewährleistet. Lediglich im äußersten Bereich des distalen Endes des Einwegschlauchs, also wo die Fixiereinrichtung für die Endoskopspitze , die Befestigungseinrichtung für den Zangenkopf und der Zangenkopf selbst vorgesehen sind, ist der Einwegschlauch vergleichsweise steif. Somit wird im Falle der genannten Inversion dieser äußerste, relativ steife Bereich des Einwegschlauchs umgeschwenkt.

Hygieneprobleme, die bei herkömmlichen Biopsiezangen im Zusammenhang mit einer unzureichenden Reinigung auftreten können, werden durch das erfindungsgemäße Einwegsystem unterbunden, da dieses nicht mehrfach verwendet wird, sondern nach dem einmaligen Gebrauch entsorgt wird. Dies gilt sowohl für den Einwegschlauch als auch für die Einwegbiopsiezange.

Die Bezeichnungen "distal" und "proximal" beziehen sich im Zusammenhang mit der vorliegenden Erfindung auf die Perspektive der Bedienperson (behandelnder Arzt), d.h. das distale Ende des Einwegschlauchs ist das freie, von der Bedienperson weg gelegene Ende.

Weiterbildungen der Erfindung sind den abhängigen Ansprüchen, der Beschreibung sowie den beigefügten Zeichnungen zu entnehmen.

Gemäß einer Ausführungsform ist die Befestigungseinrichtung an dem Zangenkopf und/oder an dem distalen Ende des Einwegschlauchs vorgesehen. Die Befestigungseinrichtung kann somit auch teilweise am Zangenkopf und teilweise am distalen Ende des Einwegschlauchs angeordnet sein. Insbesondere ist die Befestigungseinrichtung weiter proximal als die Backen der Einwegbiopsiezange angeordnet. Auf diese Weise bleiben die Backen bei einer Betätigung der Einwegbiopsiezange bezüglich des Einwegschlauchs insbesondere ortsfest und werden beispielsweise nicht in den Einwegschlauch hineingezogen.

Bei einer bevorzugten Ausführungsform ist die Befestigungseinrichtung dazu angepasst, den Zangenkopf in der Arbeitsposition am distalen Ende des Einwegschlauchs zwar axial fest, jedoch drehbeweglich zu befestigen. Mit anderen Worten ist der Zangenkopf bei dieser Ausführungsform entlang der Längsachse des Einwegschlauchs in fester Position am distalen Ende des Einwegschlauchs befestigt (temporär oder dauerhaft). Um die Längsachse des Einwegschlauchs herum ist der Zangenkopf allerdings drehbar (durchgängig oder zumindest um einen bestimmten Drehwinkel). Somit ist die gesamte Einwegzange (einschließlich der Bowdenzugseele) innerhalb des Arbeitskanals drehbar gelagert. Beispielsweise kann an dem einen Teil (Zangenkopf oder Einwegschlauch, insbesondere am Innenumfang des Arbeitskanals) eine Ringnut vorgesehen sein, in die wenigstens ein Befestigungselement des jeweiligen anderen Teils (Einwegschlauch oder Zangenkopf) eingreift, beispielsweise ein oder mehrere Befestigungszapfen, Befestigungsnasen oder Befestigungsstege.

Hierdurch ist es möglich, die Ausrichtung (d.h. die Winkelstellung) der Zangenbacken auch in der Arbeitsposition noch zu ändern, um eine Anpassung an die Umgebung (für ein verbessertes Erfassen der zu entnehmenden Gewebeprobe) und eine bessere Beobachtbarkeit (mittels des Beobachtungskanals des Endoskops) zu gestatten. Beispielsweise kann der Benutzer vom proximalen Ende des Einwegschlauchs aus den Zangenkopf durch Verdrehen der Bowdenzugseele drehen. Insbesondere im Falle der genannten Inversion ist dies von Vorteil.

Nach einer vorteilhaften Ausführungsform ist die Befestigungseinrichtung dazu angepasst, in der genannten Arbeitsposition einen Rastschluss (d.h. eine Schnappverbindung) zwischen dem distalen Ende des Einwegschlauchs und dem Zangenkopf herzustellen, wobei an dem distalen Ende des Einwegschlauchs wenigstens ein Rastvorsprung und an dem Zangenkopf wenigstens eine zugeordnete Rastvertiefung vorgesehen sind oder umgekehrt. Der Rastschluss kann insbesondere durch eine nach innen gerichtete, ringförmige Einschnürung oder durch mehrere Nasen am Ende und/oder an der Innenseite des Einwegschlauchs gebildet werden, welche mit einer zugeordneten Ringnut am Zangenkopf zusammenwirkt bzw. zusammenwirken. Durch den Rastschluss wird sowohl eine proximale als auch distale Bewegung der Backen des Zangenkopfes relativ zum Einwegschlauch unterbunden.

In einer Weiterbildung ist der genannte Rastvorsprung ein integraler Abschnitt des Einwegschlauchs oder des Zangenkopfes, der aufgrund von Eigenelastizität in Richtung einer Eingriffsstellung vorgespannt ist, um in der Eingriffsstellung in die genannte Rastvertiefung einzugreifen. Alternativ ist der genannte Rastvorsprung ein separates Element, das an dem Einwegschlauch oder dem Zangenkopf beweglich gelagert und mittels einer Vorspanneinrichtung (z.B. Federelement) in Richtung einer Eingriffsstellung vorgespannt ist. Bei dem Rastvorsprung kann es sich beispielsweise um einen radial nach außen vorgespannten Arretierschirm oder zumindest einen Arretierarm des Zangenkopfes handeln. Erreicht die Einwegbiopsiezange die Arbeitsposition, so kann sich der Rastvorsprung z.B. entspannen und mit der zugeordneten Rastvertiefung verrasten. Die Einwegbiopsiezange steht damit mit dem Einwegschlauch in Wirkverbindung.

Gemäß einer weiteren Ausführungsform ist die Befestigungseinrichtung dazu angepasst, in der genannten Arbeitsposition eine magnetische Kopplung zwischen dem distalen Ende des Einwegschlauchs und dem Zangenkopf herzustellen, wobei an dem distalen Ende des Einwegschlauchs wenigstens ein Permanentmagnet und an dem Zangenkopf wenigstens ein zugeordnetes magnetisches Gegenelement vorgesehen sind oder umgekehrt. Das Gegenelement kann dabei paramagnetisch und/oder magnetisierbar sein. Dadurch kann ein temporäres Befestigen der Einwegbiopsiezange an dem Einwegschlauch erreicht werden.

Nach einer weiteren Ausführungsform ist die Befestigungseinrichtung dazu angepasst, bei Beaufschlagung eines Zangenkopfes mit einer vorbestimmten Kraft ausgehend von der genannten Arbeitsposition den Zangenkopf von dem distalen Ende des Einwegschlauchs freizugeben. Bei der vorbestimmten Kraft kann es sich um eine bezüglich der Längsachse des Einwegschlauchs axiale Kraft handeln, aufgrund derer ab einer bestimmten Schwelle die temporäre Befestigung der Einwegbiopsiezange am Einwegschlauch gelöst werden kann. Die Kraftübertragung auf den Zangenkopf kann beispielsweise mittels der Bowdenzugseele erfolgen. Insbesondere aufgrund einer entsprechenden Formgebung einer Rastnase kann das Lösen des Zangenkopfes beispielsweise nur bei Krafteinwirkung in proximaler Richtung erfolgen. Bei einer Krafteinwirkung in distaler Richtung kann der Zangenkopf weiterhin am distalen Ende des Einwegschlauchs fixiert sein. Aufgrund einer derartigen Lösbarkeit des Zangenkopfes von dem distalen Ende des Einwegschlauchs ist es insbesondere möglich, mehrere verschiedene Einwegbiopsiezangen nacheinander in den Einwegschlauch einzuführen und zur Probenentnahme an verschiedenen Positionen innerhalb des Körpers des Patienten zu verwenden, während der Einwegschlauch selbst während des Wechsels der Einwegbiopsiezangen im Körper verbleibt.

Alternativ zu einer temporären Befestigung ist auch eine permanente Befestigung denkbar, wobei die Befestigungsmittel beider Varianten vorzugsweise derart ausgebildet sind, dass sie die Sicht des am Einwegschlauch angeordneten Endoskops nicht beeinträchtigen.

Gemäß einer Ausführungsform weist die Befestigungseinrichtung zum permanenten Befestigen des Zangenkopfes am distalen Ende des Einwegschlauchs ein stoffschlüssiges, formschlüssiges und/oder kraftschlüssiges Verbundmittel auf. Das stoffschlüssige Verbundmittel kann beispielsweise ein Klebemittel oder eine Schweißverbindung umfassen, um eine starre Verbindung herzustellen. Das formschlüssige Verbundmittel kann beispielsweise eine Bördelung aufweisen. Das kraftschlüssige Verbundmittel kann beispielsweise eine Schrumpfverbindung, eine Pressverbindung oder einen Klemmsitz umfassen. Der Zangenkopf der Einwegbiopsiezange kann somit bereits im Auslieferungszustand des Einwegsystems mit dem Einwegschlauch dauerhaft verbunden sein. Die Befestigungseinrichtung kann bei manchen Ausführungsformen in der Arbeitsposition z.B. zumindest abschnittsweise radial über den Innenradius des Arbeitskanals hervorragen. Ein Zurückziehen der Einwegbiopsiezange aus dem Arbeitskanal wird dadurch unterbunden.

Gemäß einer besonders vorteilhaften Ausführungsform weist das Einwegsystem ferner eine Einführröhre auf, die ausgehend von dem proximalen Ende des Einwegschlauchs gemeinsam mit der Biopsiezange in den Arbeitskanal einführbar ist, um den Zangenkopf in die Arbeitsposition zu bringen, wobei die Einführröhre in dem eingeführten Zustand die Bowdenzugseele umgibt, und wobei die Einführröhre vor der bestimmungsgemäßen Benutzung der Einwegbiopsiezange wieder aus dem Arbeitskanal nach proximal entfernt werden kann, während der Zangenkopf sich in der Arbeitsposition befindet. Die Einführröhre ist somit mit einem Trokar vergleichbar, welcher ebenfalls wieder entnommen werden kann. Eine derartige Einführröhre kann die Bowdenzugseele für ein Versetzen des Zangenkopfes entlang des Arbeitskanals bis zu der Arbeitsposition am distalen Ende des Einwegschlauchs stabilisieren. Die Einführröhre ist jedoch im Unterschied zu der bereits genannten Führungshülle einer herkömmlichen Biopsiezange nicht für die bestimmungsgemäße Benutzung der Einwegbiopsiezange erforderlich. Die Einführröhre kann vorzugsweise ein Kunststoffmaterial umfassen und/oder flexibel sein und z.B. als Schlauch, Rohr oder Tubus ausgebildet sein.

Sofern - wie bereits erläutert - der Zangenkopf am distalen Ende des Einwegschlauchs drehbeweglich befestigt ist, kann die genannte Einführröhre auch zum Verdrehen des Zangenkopfes vom proximalen Ende des Einwegschlauchs aus verwendet werden. Hierfür kann an dem distalen Ende der Einführröhre ein Eingriffsmittel (z.B. eine Verzahnung) vorgesehen sein, das mit einem zugeordneten Gegeneingriffsmittel (z.B. passende Gegenverzahnung) an dem Zangenkopf zusammenwirkt, um die Übertragung einer Drehbewegung zu gestatten.

Sofern alternativ zu einer derartigen Einführröhre die Einwegbiopsiezange eine Führungshülle aufweist, die dauerhaft mit dem Zangenkopf verbunden ist, so besteht die Führungshülle vorzugsweise aus einem weichen und dünnen Kunststoff.

Nach einer weiteren Ausführungsform weist das Einwegsystem ferner eine Betätigungseinrichtung zum Versetzen der Bowdenzugseele relativ zu dem Einwegschlauch auf, wobei die Betätigungseinrichtung wenigstens einen Gehäuseabschnitt aufweist, der an einer Koppeleinrichtung am proximalen Ende des Einwegschlauchs befestigbar ist, und wobei die Betätigungseinrichtung ferner wenigstens einen Antriebsabschnitt aufweist, der relativ zu dem Gehäuseabschnitt beweglich gelagert ist und der am proximalen Ende der Bowdenzugseele befestigbar ist. Durch manuelles Versetzen des Antriebsabschnitts relativ zu dem Gehäuseabschnitt können somit die Backen der Einwegbiopsiezange wahlweise geöffnet oder geschlossen werden.

Die Koppeleinrichtung zum Befestigen des Gehäuseabschnitts der Betätigungseinrichtung am proximalen Ende des Arbeitskanals des Einwegschlauchs kann beispielsweise ein Gewinde umfassen, das insbesondere nach Art eines Bajonettverschlusses wirken kann. Alternativ oder zusätzlich ist auch eine Rastverbindung denkbar. Die Befestigung des genannten Antriebsabschnitts der Betätigungseinrichtung an der Bowdenzugseele kann insbesondere mit Hilfe eines Klemmsitzes erfolgen, beispielsweise über ein Einlegen der Bowdenzugseele in eine Nut und ein einfaches Überklappen eines Klemmhebels. Die Betätigungseinrichtung kann insbesondere als Mehrwegteil ausgebildet sein, an dem die Bowdenzugseele und der Einwegschlauch lösbar befestigt werden können. Somit kann die Betätigungseinrichtung, die wesentlich einfacher als der Einwegschlauch zuverlässig gereinigt werden kann, mehrfach benutzt werden. Alternativ kann jedoch auch die Betätigungseinrichtung als Einwegteil ausgebildet sein.

Gemäß einer vorteilhaften Ausführungsform weist die Bowdenzugseele der Einwegbiopsiezange eine Seele aus Metall und (zumindest abschnittsweise) einen hiermit fest verbundenen Mantel aus Kunststoff auf. Eine Ummantelung aus Kunststoff kann ein unerwünschtes Einschneiden der Bowdenzugseele in den den Arbeitskanal umfänglich begrenzenden Einwegschlauch verhindern, insbesondere bei starken Krümmungen wie der genannten Inversion. Dies kann sich als wichtig erweisen, wenn zum Führen der Bowdenzugseele - wie bereits erläutert - keine herkömmliche Führungshülle verwendet wird sondern diese durch den Arbeitskanal des Einwegschlauchs ersetzt ist.

Gemäß einer weiteren Ausführungsform weisen die Endoskopbefestigungsmittel eine Fixiereinrichtung auf, durch die der Einwegschlauch am distalen Ende des Endoskops drehfest und axial fixierbar ist, wobei die Endoskopbefestigungsmittel ferner wenigstens eine Führungseinrichtung aufweisen, durch die der Einwegschlauch an einem jeweiligen vom distalen Ende des Endoskops beabstandeten Abschnitt des Endoskops verschieblich befestigbar ist. Die Verbindung zwischen dem Endoskop und dem Einwegschlauch wird somit durch Endoskopbefestigungsmittel hergestellt, die einerseits eine Fixiereinrichtung und andererseits eine Führungseinrichtung umfassen. Die Fixiereinrichtung kann eine feste Verbindung zwischen dem Endoskop und dem Einwegschlauch herstellen, wodurch Relativbewegungen zwischen dem distalen Ende des Endoskops und dem Einwegschlauch unterbunden werden. Hierdurch ist gewährleistet, dass eine in der Umgebung der Fixiereinrichtung angeordnete Austrittsöffnung des Arbeitskanals im Einsatz des Endoskops und insbesondere auch bei einer Krümmung des Endoskops (z.B. aufgrund einer Betätigung eines Bowdenzugs) ihre Position relativ zu dem distalen Ende des Endoskops und der dort vorgesehenen Optik stets beibehält. Somit ist eine definierte Position einer seitlichen oder axialen Öffnung des Arbeitskanals relativ zu dem distalen Ende des Endoskops sichergestellt, was beispielsweise für das Absaugen von Sekreten oder für eine Probenentnahme mittels der Einwegbiopsiezange wichtig ist, wenn zugleich eine Beobachtung mittels des Endoskops erfolgen soll.

Demgegenüber erlaubt die Führungseinrichtung eine Relativbewegung zwischen dem Endoskop und dem Einwegschlauch. Dies ermöglicht Ausgleichsbewegungen in Längsrichtung (längsverschiebliche Befestigung) und/oder in Umfangsrichtung des Einwegschlauchs (drehbewegliche Befestigung), die aufgrund von während der Benutzung auftretenden Biegungen oder Krümmungen entlang der Einheit aus Endoskop und Einwegschlauch erforderlich sind. Darüber hinaus wird das Befestigen des Endoskops vereinfacht, da eine kraftschlüssige Fixierung in der Regel nur an einem Punkt des Endoskops - nämlich an dessen distalem Ende - erfolgt.

Nach einer weiteren Ausführungsform umgibt der Einwegschlauch zusätzlich wenigstens einen Spülkanal, wobei der Einwegschlauch an seinem distalen Ende eine Austrittsöffnung des Spülkanals aufweist. Die Austrittsöffnung kann bezüglich der Fixiereinrichtung für das distale Ende des Endoskops nach distal versetzt angeordnet sein. Somit ist beispielsweise sichergestellt, dass eine am distalen Ende des Endoskops befindliche Optik mittels einer durch den Spülkanal geführten Spülflüssigkeit stets zuverlässig gespült werden kann. Der Einwegschlauch umgibt in diesem Fall also zumindest zwei Kanäle, einen Arbeitskanal und einen Spülkanal. Die Austrittsöffnung des Spülkanals und die Öffnung des Arbeitskanals sind in einem Abschnitt des Einwegschlauchs angeordnet, der in distaler Richtung des Einwegschlauchs über die Fixiereinrichtung für das distale Ende des Endoskops hinausragt, wodurch ein effizientes Spülen einer am distalen Ende des Endoskops angeordneten Optik mittels einer aus der Austrittsöffnung des Spülkanals austretenden Spülflüssigkeit möglich ist. Außerdem können mittels des Arbeitskanals durchgeführte Tätigkeiten visuell durch das Endoskop kontrolliert werden. Gemäß einer Ausführungsform umfasst der Einwegschlauch zwei Spülkanäle, die bezüglich einer Mittenebene des Einwegschlauchs exzentrisch sowie symmetrisch zueinander angeordnet sind.

Alternativ oder zusätzlich weist der Einwegschlauch an seinem distalen Ende eine Abdeckung auf, die den Arbeitskanal am distalen Ende des Einwegschlauchs abdichtet und die dazu ausgebildet ist, durch Krafteinwirkung mittels der in den Arbeitskanal eingeführten Einwegbiopsiezange geöffnet zu werden. Sobald also das Endoskop mitsamt dem Einwegsystem innerhalb des Körpers des Patienten an die gewünschte Untersuchungs- oder Behandlungsstelle gebracht worden ist (was mittels des optischen Kanals des Endoskops verifiziert werden kann), kann die den Arbeitskanal bis zu diesem Zeitpunkt abdichtende Abdeckung geöffnet werden, nämlich indem mittels der an der Rückseite der Abdeckung anliegenden Einwegbiopsiezange eine gewisse Kraft bzw. ein gewisser Druck auf die Abdeckung ausgeübt wird. Hierdurch reißt oder platzt die Abdeckung auf, oder sie löst sich teilweise oder vollständig von dem Einwegschlauch, so dass die Einwegbiopsiezange am distalen Ende des Einwegschlauchs aus dem Arbeitskanal herausgeführt werden kann. Nach dem Öffnen der Abdeckung kann unter Beobachtung mittels des optischen Kanals des Endoskops die gewünschte Probenentnahme erfolgen. Sobald beispielsweise eine geeignete Probe mittels der Einwegbiopsiezange erfasst worden ist, kann die Einwegbiopsiezange durch den Arbeitskanal des Einwegschlauchs hindurch vollständig dem Körper des Patienten entnommen werden, während das Endoskop und der hierin befestigte Einwegschlauch noch ihre Position im Körper des Patienten im Wesentlichen beibehalten.

Erst zu diesem Zeitpunkt der Entnahme der Biopsiezange dringen wesentliche Mengen der an der Untersuchungsstelle vorhandenen Körperflüssigkeit in den Arbeitskanal ein. Dies ist jedoch unkritisch, da diese Körperflüssigkeit ohnehin der Umgebung der entnommenen Probe entstammt. Durch die genannte Abdeckung wird jedoch vermieden, dass bereits während des Einführens des Einwegschlauchs in den Körper des Patienten (insbesondere entlang des Schlunds, des Rachens, des Ösophagus und/oder des Magens) Flüssigkeit aus anderen Körperbereichen als der beabsichtigten Untersuchungsstelle in den Arbeitskanal gelangt. Eine derartige Körperflüssigkeit könnte jedoch die nachfolgend in den Arbeitskanal eingeführte Einwegbiopsiezange oder eine nachfolgend durch den Arbeitskanal entnommene Probe verunreinigen. Der Arbeitskanal bleibt somit während des Einführens des Einwegschlauchs in den Körper des Patienten bis zu dem Durchstoßen oder sonstigen Öffnen der Abdeckung mittels der Einwegbiopsiezange steril. Ein weiterer Vorteil ist darin zu sehen, dass die Sterilität für den Benutzer anhand des Zustands der Abdeckung (noch nicht geöffnet) auch leicht erkennbar ist, d.h. der Benutzer und der Patient können ohne weiteres verifizieren, dass das Einwegsystem nicht bereits zu einem früheren Zeitpunkt benutzt worden ist. Durch die Vermeidung einer Kontamination des Arbeitskanals und die Erkennbarkeit einer früheren Benutzung des Einwegsystems ergeben sich also eine erhöhte Diagnosesicherheit und ein verbesserter Hygienegrad.

Die Erfindung betrifft zudem ein Verfahren zum Konfigurieren eines Einwegsystems der erläuterten Art für eine nachfolgende bestimmungsgemäße Benutzung (Endoskopie und Biopsie), mit zumindest den folgenden Schritten: Einführen der Einwegbiopsiezange in den Arbeitskanal des Einwegschlauchs von dessen proximalem Ende aus, bis der Zangenkopf das distale Ende des Einwegschlauchs erreicht, und Befestigen des Zangenkopfes am distalen Ende des Einwegschlauchs in der Arbeitsposition. Der Zangenkopf wird somit in der Arbeitsposition fixiert, um eine relative Bewegung zwischen der Bowdenzugseele und dem Einwegschlauch und/oder eine proximale Bewegung des Zangenkopfes zu unterbinden.

Nach einer Weiterbildung dieses Verfahrens weist das Einwegsystem die genannte Einführröhre auf, wobei während des Schritts des Einführens der Einwegbiopsiezange in den Arbeitskanal des Einwegschlauchs die Einführröhre die Bowdenzugseele der Einwegbiopsiezange umgibt, und wobei nach dem Schritt des Einführens der Einwegbiopsiezange in den Arbeitskanal des Einwegschlauchs die Einführröhre wieder aus dem Arbeitskanal nach proximal entfernt wird. Die Einführröhre stabilisiert somit die Bowdenzugseele während des Einführens in den Arbeitskanal. Die Einführröhre wird vorzugsweise erst nach dem Schritt des Befestigens des Zangenkopfes in der Arbeitsposition entfernt. Nach dem Einführen der Einwegbiopsiezange in den Einwegschlauch kann die Einführröhre also auch das Versetzen des Zangenkopfes in die Arbeitsposition am distalen Ende des Einwegschlauchs unterstützen, insbesondere wenn die genannte Befestigungseinrichtung einen Rastschluss erzeugen soll. Vorzugsweise wird die Einführröhre vor der bestimmungsgemäßen Benutzung der Einwegbiopsiezange wieder aus dem Arbeitskanal herausgezogen. Alternativ ist auch denkbar, die Einführröhre nur teilweise nach proximal zurückzuziehen, oder die Einführröhre wird gänzlich im Einwegschlauch belassen und dient somit während einer nachfolgenden Benutzung der Einwegbiopsiezange als Führungshülle für die Bowdenzugseele.

Gemäß einer weiteren Ausführungsform weist das Einwegsystem die genannte Betätigungseinrichtung zum Versetzen der Bowdenzugseele relativ zu dem Einwegschlauch auf, wobei das Konfigurationsverfahren zusätzlich die folgenden Schritte umfasst, die nach dem Schritt des Befestigens des Zangenkopfes in der Arbeitsposition durchgeführt werden: Befestigen des Gehäuseabschnitts der Betätigungseinrichtung am proximalen Ende des Einwegschlauchs, und Befestigen des Antriebsabschnitts der Betätigungseinrichtung am proximalen Ende der Bowdenzugseele. Die Durchführung dieser weiteren Schritte erfolgt vorzugsweise nach Entfernen der Einführröhre, sofern eine solche benutzt wird.

Nach einer weiteren Ausführungsform umfasst das Einwegsystem zusätzlich zu der genannten einen Einwegbiopsiezange wenigstens eine weitere Einwegbiopsiezange, die einen Zangenkopf mit wenigstens zwei beweglichen Backen und eine Bowdenzugseele zum Betätigen der Backen aufweist, wobei das Konfigurationsverfahren zusätzlich die folgenden Schritte umfasst: Lösen des Zangenkopfes der genannten einen Einwegbiopsiezange vom distalen Ende des Einwegschlauchs und Herausziehen der genannten einen Einwegbiopsiezange aus dem Arbeitskanal des Einwegschlauchs; Einführen der genannten weiteren Einwegbiopsiezange in den Arbeitskanal des Einwegschlauchs von dessen proximalem Ende aus, bis der Zangenkopf der genannten weiteren Einwegbiopsiezange das distale Ende des Einwegschlauchs erreicht; und Befestigen des Zangenkopfes der genannten weiteren Einwegbiopsiezange am distalen Ende des Einwegschlauchs in der Arbeitsposition. Dies ermöglicht insbesondere einen Wechsel der Einwegbiopsiezange während der Behandlung, d.h. während der Einwegschlauch im Körper des Patienten verbleibt, beispielsweise um mittels einer entsprechenden Anzahl von verschiedenen Einwegbiopsiezangen nacheinander mehrere Gewebeproben an verschiedenen Stellen im Körper zu entnehmen. Hierzu kann vor dem Lösen des Zangenkopfes der genannten einen Einwegbiopsiezange die genannte Betätigungseinrichtung entfernt werden, die nach dem Einführen und Befestigen der weiteren Einwegbiopsiezange wieder am Einwegschlauch angebracht werden kann.

Bei dieser Ausführungsform mit wenigstens einer weiteren Einwegbiopsiezange kann das Einwegsystem eine Einführröhre aufweisen, insbesondere die bereits genannte Einführröhre (für die genannte eine Einwegbiopsiezange) oder eine weitere Einführröhre. Während des Schritts des Einführens der genannten weiteren Einwegbiopsiezange in den Arbeitskanal des Einwegschlauchs umgibt die Einführröhre die Bowdenzugseele der genannten weiteren Einwegbiopsiezange, um die Bowdenzugseele während des Einführens in den Arbeitskanal zu stabilisieren und den Zangenkopf mittels der Einführröhre nach vorne drücken zu können. Nach dem Schritt des Einführens der genannten weiteren Einwegbiopsiezange in den Arbeitskanal wird die Einführröhre wieder aus dem Arbeitskanal nach proximal entfernt, wobei dies vorzugsweise erst nach dem Schritt des Befestigens des Zangenkopfes in der Arbeitsposition und noch vor der bestimmungsgemäßen Benutzung der genannten weiteren Einwegbiopsiezange geschieht.

Die Erfindung wird im Folgenden beispielhaft unter Bezugnahme auf die Zeichnungen erläutert. Es zeigen:
- Fig. 1a: eine schematische Darstellung eines Endoskopie- und Biopsie-Einwegsystems,
- Fig. 1b: eine um 90° gedrehte Ansicht des Einwegsystems gemäß Fig. 1a,
- Fig. 2: einen Schnitt durch das Einwegsystem gemäß Fig. 1a entlang der Ebene A-A,
- Fig. 3: eine Detailansicht des Bereichs B des Einwegsystems gemäß Fig. 1a vor Erreichen der Arbeitsposition,
- Fig. 4: eine Detailansicht des Bereichs B des Einwegsystems gemäß Fig. 1a in der Arbeitsposition mit einer ersten Ausführungsform einer Befestigungseinrichtung,
- Fig. 5: eine Detailansicht des Bereichs B des Einwegsystems gemäß Fig. 1a in einer Arbeitsposition mit einer zweiten Ausführungsform einer Befestigungseinrichtung,
- Fig. 6: eine Detailansicht des Bereichs B des Einwegsystems gemäß Fig. 1a in einer Arbeitsposition mit einer dritten Ausführungsform einer Befestigungseinrichtung,
- Fig. 7: eine Detailansicht des Bereichs B des Einwegsystems gemäß Fig. 1a in einer Arbeitsposition mit einer vierten Ausführungsform einer Befestigungseinrichtung, und
- Fig. 8: eine Detailansicht des proximalen Endes eines Einwegsystems gemäß Fig. 1a und 1b mit einer Betätigungseinrichtung.

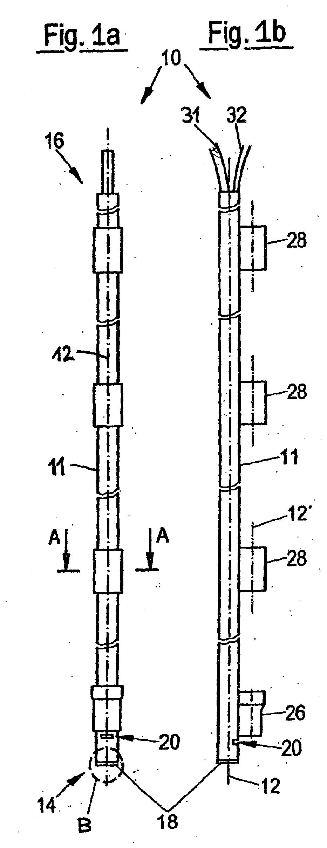

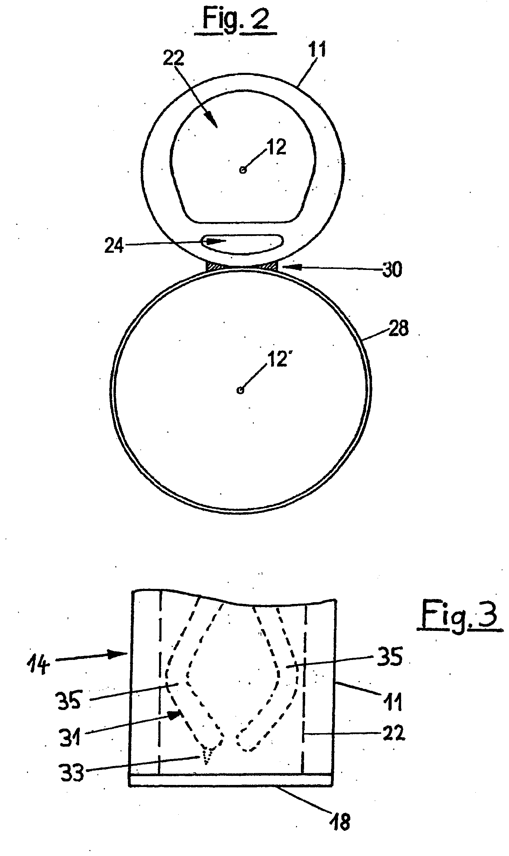

Fig. 1a und Fig. 1b zeigen ein Endoskopie- und Biopsie-Einwegsystem 10 mit einem flexiblen Einwegschlauch 11, wobei die Fig. 1b eine gegenüber der Fig. 1a um 90° um eine Längsachse 12 verdrehte Ansicht darstellt. Der Einwegschlauch 11 weist ein distales Ende 14 und ein proximales Ende 16 auf. Nahe dem distalen Ende 14 des Einwegschlauchs 11 ist eine als Fixiermanschette 26 ausgebildete Fixiereinrichtung an dem Einwegschlauch 11 befestigt. Die Fixiermanschette 26 dient zum drehfesten und axial festen Fixieren des distalen Endes eines in die Fixiermanschette 26 einzuführenden Endoskops (nicht gezeigt). Die Fixiermanschette 26 ist weiter proximal angeordnet als eine optionale, stirnseitige Abdeckung 18 des Einwegschlauchs 11 und eine seitliche Austrittsöffnung 20. Die Abdeckung 18 verschließt einen Arbeitskanal 22, und die Austrittsöffnung 20 steht mit einem Spülkanal 24 des Einwegschlauchs 11 in Verbindung (vgl. Fig. 2).

Durch die räumliche Nähe zu der Fixiermanschette 26 und die schlitzartige Formgebung der als Düse wirkenden Austrittsöffnung 20 kann Spülflüssigkeit auf die Optik eines in der Fixiermanschette 26 gehaltenen Endoskops gesprüht werden, um diese zu reinigen und beispielsweise störendes Sekret zu entfernen. Alternativ hierzu kann auch Luft durch den Spülkanal 24 geführt werden, um nicht nur die Optik des Endoskops frei zu blasen, sondern um durch Luftinsufflation auch den zu untersuchenden Hohlraum aufzublasen, so dass eine Wand beispielsweise des Magens besser beobachtet werden kann.

Zusätzlich zu der Fixiermanschette 26 sind in regelmäßigen Abständen mehrere Führungsschlaufen 28 an dem Einwegschlauch 11 des Einwegsystems 10 angebracht, welche als Führungseinrichtungen dienen. Die Fixiermanschette 26 und die Führungsschlaufen 28 besitzen bei geradlinig ausgerichtetem Einwegschlauch 11 eine gemeinsame Längsachse 12'.

Fig. 2 verdeutlicht den Aufbau des Einwegsystems 10 anhand eines Querschnitts entlang der Ebene A-A gemäß Fig. 1a. Im oberen Teil der Fig. 2 ist der Querschnitt des Einwegschlauchs 11 zu sehen. Der Einwegschlauch umfasst - wie vorstehend erläutert - den Arbeitskanal 22 und den Spülkanal 24. Zur besseren Nutzung der Querschnittsfläche des Einwegschlauchs 11 sind bei dem hier gezeigten Ausführungsbeispiel weder der Arbeitskanal 22 noch der Spülkanal 24 kreisförmig ausgebildet. Allerdings kann der Querschnitt des Arbeitskanals 22 auch kreisförmig sein. Der Spülkanal 24 liegt zwischen dem Arbeitskanal 22 und den Führungsschlaufen 28. Es können auch mehrere Spülkanäle 24 vorgesehen sein, z.B. zwei mittensymmetrisch angeordnete Spülkanäle 24.

Im unteren Teil der Darstellung gemäß Fig. 2 ist der Querschnitt einer der Führungsschlaufen 28 gezeigt. Diese weist einen kreisförmigen Querschnitt auf, der zur Aufnahme des Endoskops dient. Innerhalb der Führungsschlaufe 28 kann sich das Endoskop insbesondere in Längsrichtung bewegen. Die Führungsschlaufen 28 dienen somit dazu, den Einwegschlauch 11 des Einwegsystems 10 in seitlicher Richtung in enger räumlicher Nähe zu dem Endoskop zu halten, sich allerdings nicht fest miteinander zu verbinden. Dies ist, wie vorstehend bereits erläutert, Aufgabe der Fixiermanschette 26. Die Führungsschlaufe 28 ist an den Einwegschlauch angeklebt, wie durch die Klebeverbindung 30 angedeutet ist. Die Art der Verbindung kann allerdings frei gewählt werden, beispielsweise ist alternativ eine Schweißverbindung oder eine Schrumpfverbindung möglich. Auch eine einstückige Ausführung des Einwegschlauchs 11 und der Führungsschlaufen 28 ist denkbar. Entsprechendes gilt für die Fixiermanschette 26.

Mit Bezug wiederum auf Fig. 1b ist am proximalen Ende des Einwegschlauchs 11 ein Abschnitt einer in den Arbeitskanal 22 eingeführten Einwegbiopsiezange 31 gezeigt, wie nachstehend noch näher erläutert wird. Zudem ist ein in den Spülkanal 24 mündender Verbindungsschlauch 32 gezeigt, der zur Zufuhr einer Spülflüssigkeit dient.

Der Spülkanal 24 und die zugeordnete Austrittsöffnung 20 können auch entfallen. Ebenso können die erläuterten Endoskopiebefestigungsmittel (Fixiermanschette 26 und Führungsschlaufe 28) auch andersartig ausgebildet sein.

Fig. 3 zeigt eine Detailansicht des distalen Endes 14 des Einwegschlauchs 11 (entsprechend dem Bereich B gemäß Fig. 1a) während einer Phase des Konfigurierens des Einwegsystems 10 für eine nachfolgende Benutzung. Die dort vorgesehene Abdeckung 18 dichtet den Arbeitskanal 22 (gestrichelt dargestellt) zunächst vollständig ab, so dass der Arbeitskanal 22 lediglich am proximalen Ende 16 des Einwegschlauchs 11 geöffnet ist. Die Abdeckung 18 ist in dem hier gezeigten Ausführungsbeispiel durch eine Folie gebildet, die im Vergleich zu dem Durchmesser des Einwegschlauchs 11 und auch im Vergleich zu der Wandstärke des Einwegschlauchs 11 (im Umfangsbereich der geringsten Wandstärke gemäß Fig. 2) relativ dünn ist. Die Abdeckung 18 dient dazu, den Arbeitskanal 22 während des Einführens des Einwegsystems 10 (gemeinsam mit dem Endoskop) in dem Körper des Patienten dicht zu halten, um ein vorzeitiges Eindringen von Körperflüssigkeit in den Arbeitskanal 22 zu verhindern. Nach dem Einführen des Endoskops mitsamt Einwegsystem in den Körper des Patienten wird unter Beobachtung des distalen Endes 14 des Einwegschlauchs 11 mittels des optischen Kanals des Endoskops das Endoskop in die endgültig gewünschte Position gebracht. Spätestens jetzt wird auch die Einwegbiopsiezange 31 (in Fig. 3 ebenfalls gestrichelt dargestellt) in den Arbeitskanal 22 eingeführt, bis das distale Ende der Einwegbiopsiezange 31 unmittelbar vor der Rückseite der Abdeckung 18 zu liegen kommt. Dieser Zustand kann der Bedienperson beispielsweise durch eine Längenmarkierung am proximalen Ende der Einwegbiopsiezange 31 verdeutlich werden, die mit einer zugeordneten Markierung am proximalen Ende des Einwegsystems 10 korrespondiert. Erst zu diesem Zeitpunkt wird die Abdeckung 18 des Arbeitskanals 22 geöffnet, nämlich indem mittels der Einwegbiopsiezange 31 eine vorbestimmte Kraft (z.B. Wert zwischen 0,5 N und 10 N) auf die Abdeckung 18 ausgeübt wird. Um das Öffnen der Abdeckung 18 mittels der Einwegbiopsiezange 31 zu erleichtern, kann die Biopsiezange 31 an ihrem distalen Ende einen Aufreißdorn 33 aufweisen, wie in Fig. 3 ebenfalls gezeigt ist. Der Aufreißdorn 33 ist an einer der Backen 35 der Einwegbiopsiezange 31 angeordnet. Ferner kann die Abdeckung 18 eine beispielsweise längliche, sich diametral erstreckende Sollbruchstelle aufweisen.

Ein besonderer Vorteil der Abdeckung 18 besteht auch darin, dass nach dem Öffnen die verbleibenden Abschnitte der Abdeckung 18 (z.B. die Begrenzungen des entstandene Schlitzes) als Dichtlippen für die Einwegbiopsiezange 31 wirken können, so dass die Abdeckung 18 trotz der gebildeten Öffnung ein Einströmen von Körperflüssigkeit in den Arbeitskanal 22 (so genannter Reflux) weitgehend verhindert wird.

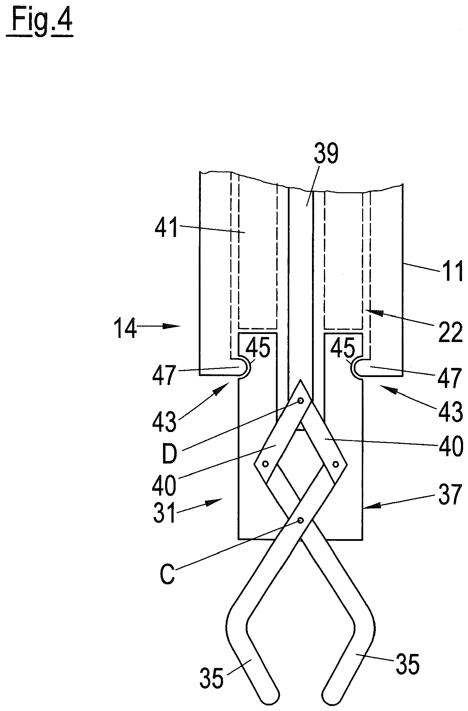

Fig. 4 zeigt eine Detailansicht des distalen Endes 14 des Einwegschlauchs 11 (entsprechend dem Bereich B gemäß Fig. 1a) während einer späteren Phase des Konfigurierens des Einwegsystems 10, in der ein Zangenkopf 37 der Einwegbiopsiezange 31 eine Arbeitsposition eingenommen hat. Die Backen 35 der Einwegbiopsiezange 31 bilden einen Teil des Zangenkopfes 37 und sind an dem Zangenkopf 37 um eine gemeinsame Schwenkachse C gegenläufig schwenkbar gelagert. In der gezeigten Arbeitsposition ragen die Backen 35 und ein Teil des Zangenkopfes 37 über das distale Ende 14 des Einwegschlauchs 11 hervor.

Im Bereich des Zangenkopfes 37 ist ein Stahldraht zu erkennen, der als Bowdenzugseele 39 dient und der optional mit einem dünnen Mantel aus Kunststoff versehen sein kann. Zwei Umlenkhebel 40, die mit den Backen 35 gelenkig gekoppelt sind, sind an dem Ende der Bowdenzugseele 39 befestigt und dort um eine gemeinsame Schwenkachse D gegenläufig schwenkbar gelagert. Durch Versetzen der Bowdenzugseele 39 relativ zu dem Zangenkopf 37 können somit die Backen 35 der Einwegbiopsiezange 31 wahlweise geöffnet oder geschlossen werden.

Die Bowdenzugseele 39 ist bei dem hier gezeigten Ausführungsbeispiel in einer optionalen, schlauchartigen Einführröhre 41 angeordnet, welche insbesondere aus einem Kunststoffmaterial besteht. Die Einführröhre 41 erleichtert das Einführen der Einwegbiopsiezange 31 in den Arbeitskanal 22 des Einwegschlauchs 11 bis zu dem Erreichen der gezeigten Arbeitsposition.

Am distalen Ende 14 des Einwegschlauchs 11 ist eine Befestigungseinrichtung 43 für den Zangenkopf 37 vorgesehen, um den Zangenkopf 37 in seiner Arbeitsposition am distalen Ende 14 des Einwegschlauchs 11 festzulegen. Dies geschieht bei dem hier gezeigten Ausführungsbeispiel durch einen lösbaren Rastschluss, d.h. durch eine lösbare Schnappverbindung. Die Befestigungseinrichtung 43 umfasst hierfür eine Rastvertiefung 45 am Zangenkopf 37, welche die Form einer umlaufenden Ringnut besitzt. In die Rastvertiefung 45 des Zangenkopfes 37 kann ein rückfedernd ausgebildeter Rastvorsprung 47 des Einwegschlauchs 11 einrasten, wenn sich die Einwegbiopsiezange 31 in der gezeigten Arbeitsposition befindet. Der Rastvorsprung 47 besitzt die Form eines radial nach innen ragenden umlaufenden Stegs.

Für das Konfigurieren des Einwegsystems 10 wird also ausgehend von dem proximalen Ende 16 des Einwegschlauchs 11 die Einwegbiopsiezange 31 mit dem Zangenkopf 37 voraus durch den Arbeitskanal 22 geführt, wobei diese Bewegung mittels der Einführröhre 41 unterstützt wird. Insbesondere kann die Einführröhre 41 dazu verwendet werden, den Zangenkopf 37 nach vorne bis in die genannte Arbeitsposition zu schieben. Der Einwegschlauch 11 kann sich hierbei bereits im Körper des Patienten befinden. Die Einführbewegung der Einwegbiopsiezange 31 wird solange durchgeführt, bis die Spitze der Einwegbiopsiezange 31 am distalen Ende 14 des Einwegschlauchs 11 aus dem Arbeitskanal 22 ausgefahren ist. Hierdurch wird die in Fig. 4 gezeigte Arbeitsposition erreicht, in der ein Rastschluss zwischen dem distalen Ende 14 des Einwegschlauchs 11 und dem Zangenkopf 37 erzeugt wird, wodurch eine kontrollierte Relativbewegung zwischen dem Zangenkopf 37 und der Bowdenzugseele 39 in axialer Richtung ermöglicht wird. Da die Rastvertiefung 45 am Zangenkopf 37, mit welcher der Rastvorsprung 47 des Einwegschlauchs 11 zusammenwirkt, als eine Ringnut ausgebildet ist, kann der Zangenkopf 37 in der gezeigten Arbeitsposition erforderlichenfalls in eine geeignete Winkelstellung gedreht werden.

Nun kann zunächst die Einführröhre 41 entfernt werden. Anschließend kann am proximalen Ende des Einwegschlauchs 11 noch eine Betätigungseinrichtung angebracht werden, wie nachstehend noch erläutert wird. Nun kann mittels der Einwegbiopsiezange 31 eine gewünschte Probe entnommen werden, während gleichzeitig eine Beobachtung mittels des zugeordneten Endoskops erfolgen kann. Wird Kraft auf die Bowdenzugseele 39 ausgeübt, so schließen sich die Backen 35. Sobald eine Gewebeprobe oder dergleichen erfasst ist, kann die Einwegbiopsiezange 31 durch den Arbeitskanal 22 aus dem Körper des Patienten entnommen werden.

Durch Aufbringen einer vorbestimmten Zugkraft auf die Bowdenzugseele 39 kann der von der Befestigungseinrichtung 43 erzeugte Rastschluss wahlweise überwunden werden, so dass der Rastvorsprung 47 nicht mehr in Eingriff mit der Rastvertiefung 45 steht. Nach einem teilweisen oder vollständigen Zurückziehen der Einwegbiopsiezange 31 wird auch das Endoskop mitsamt Einwegsystem 10 aus dem Körper des Patienten zurückgezogen. Ist die Einwegbiopsiezange 31 mitsamt der Probe aus dem Arbeitskanal entnommen, kann alternativ eine neue Einwegbiopsiezange 31 in den Arbeitskanal eingeführt werden, beispielsweise um eine weitere Probe, insbesondere an einer anderen Stelle im Körper, zu entnehmen.

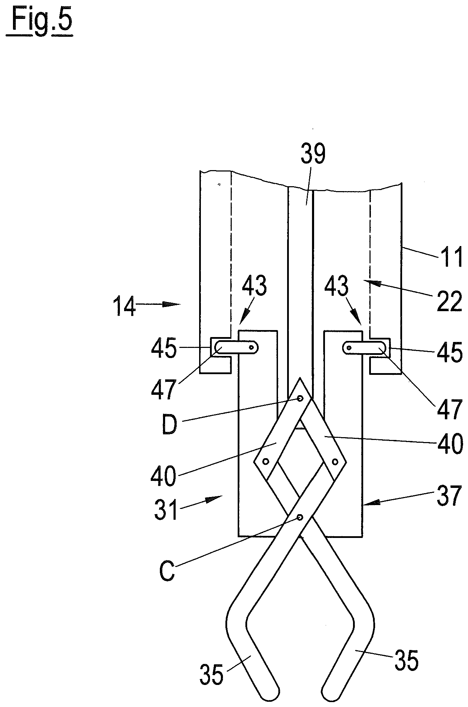

In Fig. 5 ist eine alternative Befestigungseinrichtung 43 dargestellt. Im Unterschied zu der Befestigungseinrichtung 43 gemäß Fig. 4 ist die Rastvertiefung 45 hierbei als Ringnut an der Innenseite des Einwegschlauchs 11 ausgebildet. Der Rastvorsprung 47 wird durch ein jeweiliges Eingriffselement gebildet, welches nach radial außen vorgespannt ist und in die Rastvertiefung 45 einrastet, wenn sich die Einwegbiopsiezange 31 in der Arbeitsposition befindet. Die Vorspannung kann beispielsweise mit Hilfe einer Federeinrichtung (nicht dargestellt) realisiert werden. Alternativ ist auch denkbar, den jeweiligen Rastvorsprung 47 als elastisches Element auszubilden. Zum Entnehmen der Einwegbiopsiezange 31 kann auch diese Befestigungseinrichtung 43 bei einer entsprechenden Krafteinwirkung überwunden werden.

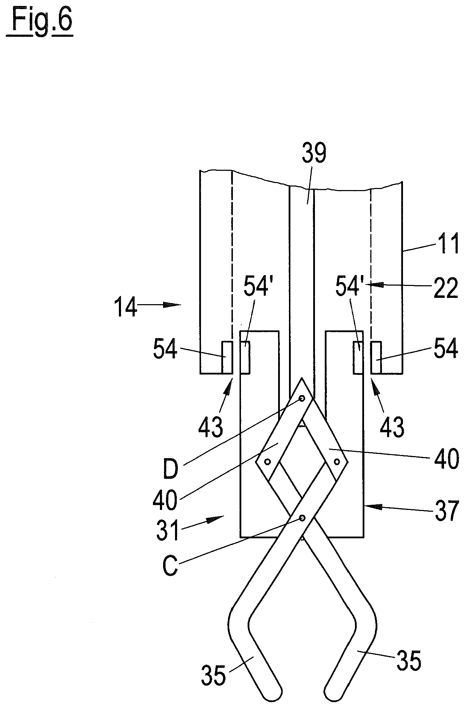

Fig. 6 zeigt ein weiteres Ausführungsbeispiel, bei dem die Befestigungseinrichtung 43 einen ringförmigen Permanentmagneten 54 an der Innenseite des Einwegschlauchs 11 umfasst. Dieser wirkt mit einem ringförmigen magnetischen Gegenelement 54' des Zangenkopfes 37 zusammen und bildet mit diesem eine lösbare magnetische Kopplung.

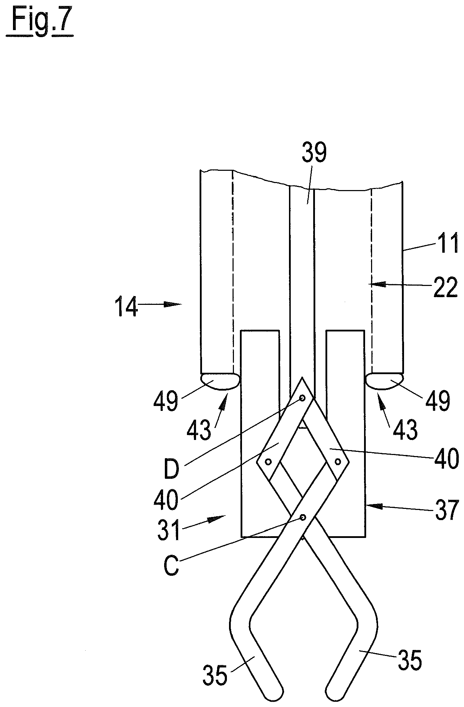

In Fig. 7 ist die Befestigungseinrichtung 43 dazu ausgebildet, den Zangenkopf 37 permanent am Einwegschlauch 11 zu befestigen. Dies kann beispielsweise mittels eines Klebemittels 49 realisiert werden, welches den Zangenkopf 37 am Einwegschlauch 11 festklebt. Denkbar wäre beispielsweise auch eine Kunststoff-Schweißverbindung oder eine Verjüngung des Einwegschlauchs 11, so dass der Zangenkopf 37 nicht gänzlich aus dem Einwegschlauch 11 austreten kann und ein dauerhafter Klemmsitz gebildet wird. Der Einwegschlauch 11 und der Zangenkopf 37 der Einwegbiopsiezange 31 können auch einstückig aufgebaut sein.

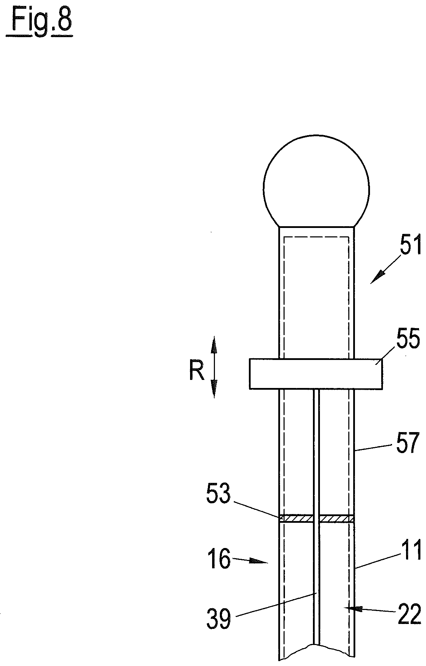

Zum Öffnen und Schließen der Backen 35 des Zangenkopfes 37 ist eine Betätigungseinrichtung 51 vorgesehen, welche in Fig. 8 schematisch dargestellt ist. Ein Gehäuseabschnitt 57 der Betätigungseinrichtung 51 ist am proximalen Ende 16 des Einwegschlauchs 11 angeordnet und mit diesem mithilfe einer als Gewinde 53 ausgebildeten Koppeleinrichtung verbunden. Die Bowdenzugseele 39 ist hingegen mit einem als Schieber ausgebildeten Antriebsabschnitt 55 der Betätigungseinrichtung 51 verbunden. Der Antriebsabschnitt 55 ist relativ zum Gehäuseabschnitt 57 beweglich. Auf diese Weise kann die Bowdenzugseele 39 relativ zum Einwegschlauch 11 wahlweise entlang einer Betätigungsrichtung R bewegt werden. Wird der Antriebsabschnitt 55 beispielsweise in distale Richtung verschoben, so öffnen sich die Backen 35 der Einwegbiopsiezange 31 und umgekehrt.

Bei dem erfindungsgemäßen Einwegsystem 10 ist somit eine unerwünscht steife Ummantelung der Bowdenzugseele 39, beispielsweise aus Metall, nicht notwendig, wodurch die Flexibilität des Einwegsystems 10 deutlich erhöht wird. Durch die Verwendung von Einwegteilen wird auch die Hygiene bei einer Endoskopie deutlich verbessert.

### Bezugszeichenliste

- 10: Endoskopie- und Biopsie-Einwegsystem
- 11: Einwegschlauch
- 12: Längsachse des Einwegschlauchs
- 12': Längsachse der Fixiermanschette und der Führungsschlaufen
- 14: distales Ende
- 16: proximales Ende
- 18: Abdeckung
- 20: Austrittsöffnung
- 22: Arbeitskanal
- 24: Spülkanal
- 26: Fixiermanschette
- 28: Führungsschlaufe
- 30: Klebeverbindung
- 31: Einwegbiopsiezange
- 32: Verbindungsschlauch
- 33: Aufreißdorn
- 35: Backe
- 37: Zangenkopf
- 39: Bowdenzugseele
- 40: Umlenkhebel
- 41: Einführröhre
- 43: Befestigungseinrichtung
- 45: Rastvertiefung
- 47: Rastvorsprung
- 49: Klebemittel
- 51: Betätigungseinrichtung
- 53: Gewinde
- 54: Permanentmagnet
- 54': magnetisches Gegenelement
- 55: Antriebsabschnitt
- 57: Gehäuseabschnitt

- R: Betätigungsrichtung
- C: Schwenkachse
- D: Schwenkachse

## Patentansprüche

1. Endoskopie- und Biopsie-Einwegsystem (10), mit:
einem Einwegschlauch (11), der zumindest einen Arbeitskanal (22) umgibt und der Endoskopbefestigungsmittel (26, 28) zum Befestigen des Einwegschlauchs (11) an einem Endoskop aufweist; und
wenigstens einer Einwegbiopsiezange (31), die in den Arbeitskanal (22) einführbar oder eingeführt ist, wobei die Einwegbiopsiezange (31) einen Zangenkopf (37) mit wenigstens zwei beweglichen Backen (35) und eine Bowdenzugseele (39) zum Betätigen der Backen (35) aufweist;
wobei eine Befestigungseinrichtung (43) zum temporären oder permanenten Befestigen des Zangenkopfes (37) am distalen Ende (14) des Einwegschlauchs (11) in einer Arbeitsposition, in der die Backen (35) des Zangenkopfes (37) über das distale Ende (14) des Einwegschlauchs (11) hervorragen, vorgesehen ist,
**dadurch gekennzeichnet, dass**
die Befestigungseinrichtung (43) an dem Zangenkopf (37) und/oder an dem distalen Ende (14) des Einwegschlauchs (11) vorgesehen ist.

2. Einwegsystem nach Anspruch 1,
wobei die Befestigungseinrichtung (43) dazu angepasst ist, den Zangenkopf (37) in der Arbeitsposition am distalen Ende (14) des Einwegschlauchs (11) axial fest, jedoch drehbeweglich zu befestigen.

3. Einwegsystem nach einem der vorstehenden Ansprüche,
wobei die Befestigungseinrichtung (43) dazu angepasst ist, in der genannten Arbeitsposition einen Rastschluss zwischen dem distalen Ende (14) des Einwegschlauchs (11) und dem Zangenkopf (37) herzustellen, wobei an dem distalen Ende (14) des Einwegschlauchs (11) wenigstens ein Rastvorsprung (47) und an dem Zangenkopf (37) wenigstens eine zugeordnete Rastvertiefung (45) vorgesehen sind oder umgekehrt.

4. Einwegsystem nach Anspruch 3,
wobei der wenigstens eine Rastvorsprung (47) ein integraler Abschnitt des Einwegschlauchs (11) oder des Zangenkopfes (37) ist, der aufgrund von Eigenelastizität in Richtung einer Eingriffsstellung vorgespannt ist; oder
wobei der wenigstens eine Rastvorsprung (47) ein separates Element ist, das an dem Einwegschlauch (11) oder dem Zangenkopf (37) beweglich gelagert und mittels einer Vorspanneinrichtung in Richtung einer Eingriffsstellung vorgespannt ist.

5. Einwegsystem nach einem der Ansprüche 1 oder 2,
wobei die Befestigungseinrichtung (43) dazu angepasst ist, in der genannten Arbeitsposition eine magnetische Kopplung zwischen dem distalen Ende des Einwegschlauchs (11) und dem Zangenkopf (37) herzustellen, wobei an dem distalen Ende (14) des Einwegschlauchs (11) wenigstens ein Permanentmagnet (54) und an dem Zangenkopf (37) wenigstens ein zugeordnetes magnetisches Gegenelement (54') vorgesehen sind oder umgekehrt.

6. Einwegsystem nach einem der vorstehenden Ansprüche,
wobei die Befestigungseinrichtung (43) dazu angepasst ist, bei Beaufschlagung des Zangenkopfes (37) mit einer vorbestimmten Kraft ausgehend von der genannten Arbeitsposition den Zangenkopf (37) von dem distalen Ende (14) des Einwegschlauchs (11) freizugeben; oder
wobei die Befestigungseinrichtung (43) zum permanenten Befestigen des Zangenkopfes (37) am distalen Ende (14) des Einwegschlauchs (11) ein stoffschlüssiges, formschlüssiges und/oder kraftschlüssiges Verbundmittel aufweist.

7. Einwegsystem nach einem der vorstehenden Ansprüche,
wobei das Einwegsystem ferner eine Einführröhre (41) aufweist, die dazu angepasst ist, ausgehend von dem proximalen Ende (16) des Einwegschlauchs (11) gemeinsam mit der Einwegbiopsiezange (31) in den Arbeitskanal (22) eingeführt zu werden, um den Zangenkopf (37) in die Arbeitsposition zu bringen, wobei die Einführröhre (41) in dem eingeführten Zustand die Bowdenzugseele (39) umgibt, und wobei die Einführröhre (41) dazu angepasst ist, aus dem Arbeitskanal (22) nach proximal entfernt zu werden, während der Zangenkopf (37) sich in der Arbeitsposition befindet;
wobei die Befestigungseinrichtung (43) vorzugsweise dazu angepasst ist, den Zangenkopf (37) in der Arbeitsposition am distalen Ende (14) des Einwegschlauchs (11) axial fest, jedoch drehbeweglich zu befestigen, wobei die Einführröhre (41) an einem distalen Ende ein Eingriffsmittel aufweist, das mit einem zugeordneten Gegeneingriffsmittel an dem Zangenkopf (37) derart zusammenwirkt, dass eine Drehbewegung von der Einführröhre (41) auf den in der Arbeitsposition befestigten Zangenkopf (37) übertragbar ist.

8. Einwegsystem nach einem der vorstehenden Ansprüche,
wobei das Einwegsystem ferner eine Betätigungseinrichtung (51) zum Versetzen der Bowdenzugseele (39) relativ zu dem Einwegschlauch (11) aufweist, wobei die Betätigungseinrichtung (51) wenigstens einen Gehäuseabschnitt (57) aufweist, der an einer Koppeleinrichtung (53) am proximalen Ende (16) des Einwegschlauchs (11) befestigbar ist, und wobei die Betätigungseinrichtung (51) ferner wenigstens einen Antriebsabschnitt (55) aufweist, der relativ zu dem Gehäuseabschnitt (57) beweglich gelagert ist und der am proximalen Ende der Bowdenzugseele (39) befestigbar ist;
und/oder
wobei die Bowdenzugseele (39) der Einwegbiopsiezange (31) eine Seele aus Metall und einen Mantel aus Kunststoff aufweist.

9. Einwegsystem nach einem der vorstehenden Ansprüche,
wobei die Endoskopbefestigungsmittel eine Fixiereinrichtung (26) aufweisen, durch die der Einwegschlauch (11) am distalen Ende (14) des Endoskops drehfest und axial fest fixierbar ist, und wobei die Endoskopbefestigungsmittel ferner wenigstens eine Führungseinrichtung (28) aufweisen, durch die der Einwegschlauch (11) an einem jeweiligen vom distalen Ende (14) des Endoskops beabstandeten Abschnitt des Endoskops verschieblich befestigbar ist; und/oder
wobei der Einwegschlauch (11) zusätzlich wenigstens einen Spülkanal (24) umgibt, wobei der Einwegschlauch (11) an seinem distalen Ende (14) eine Austrittsöffnung (20) des Spülkanals (24) aufweist; und/oder
wobei der Einwegschlauch (11) an seinem distalen Ende (14) eine Abdeckung (18) aufweist, die den Arbeitskanal (22) am distalen Ende (14) des Einwegschlauchs (11) abdichtet und die dazu ausgebildet ist, durch Krafteinwirkung mittels der in den Arbeitskanal (22) eingeführten Einwegbiopsiezange (31) geöffnet zu werden.

10. Verfahren zum Konfigurieren eines Einwegsystems nach einem der vorstehenden Ansprüche, mit den Schritten:
Einführen der Einwegbiopsiezange (31) in den Arbeitskanal (22) des Einwegschlauchs (11) von dessen proximalem Ende (16) aus, bis der Zangenkopf (37) das distale Ende (14) des Einwegschlauchs (11) erreicht; und
Befestigen des Zangenkopfes (37) am distalen Ende (14) des Einwegschlauchs (11) in der Arbeitsposition.

11. Verfahren nach Anspruch 10,
wobei das Einwegsystem eine Einführröhre (41) aufweist;
wobei während des Schritts des Einführens der Einwegbiopsiezange (31) in den Arbeitskanal (22) des Einwegschlauchs (11) die Einführröhre (41) die Bowdenzugseele (39) der Einwegbiopsiezange (31) umgibt; und
wobei nach dem Schritt des Einführens der Einwegbiopsiezange (31) in den Arbeitskanal (22) des Einwegschlauchs (11) die Einführröhre (41) aus dem Arbeitskanal (22) nach proximal entfernt wird.

12. Verfahren nach Anspruch 10 oder 11,
wobei das Einwegsystem eine Betätigungseinrichtung (51) zum Versetzen der Bowdenzugseele (39) relativ zu dem Einwegschlauch (11) aufweist, wobei die Betätigungseinrichtung (51) wenigstens einen Gehäuseabschnitt (57) und wenigstens einen Antriebsabschnitt (55) aufweist, der relativ zu dem Gehäuseabschnitt (57) beweglich gelagert ist;
mit den zusätzlichen Schritten, die nach dem Schritt des Befestigens des Zangenkopfes (37) in der Arbeitsposition durchgeführt werden:
Befestigen des Gehäuseabschnitts (57) der Betätigungseinrichtung (51) am proximalen Ende (16) des Einwegschlauchs (11); und Befestigen des Antriebsabschnitts (55) der Betätigungseinrichtung (51) am proximalen Ende der Bowdenzugseele (39).

13. Verfahren nach einem der Ansprüche 10 bis 12,
wobei das Einwegsystem wenigstens eine weitere Einwegbiopsiezange umfasst, die einen Zangenkopf mit wenigstens zwei beweglichen Backen und eine Bowdenzugseele zum Betätigen der Backen aufweist;
mit den zusätzlichen Schritten:
Lösen des Zangenkopfes (37) der genannten einen Einwegbiopsiezange (31) vom distalen Ende (14) des Einwegschlauchs (11) und Herausziehen der genannten einen Einwegbiopsiezange (31) aus dem Arbeitskanal (22) des Einwegschlauchs (11);
Einführen der genannten weiteren Einwegbiopsiezange in den Arbeitskanal (22) des Einwegschlauchs (11) von dessen proximalem Ende (16) aus, bis der Zangenkopf der genannten weiteren Einwegbiopsiezange das distale Ende (14) des Einwegschlauchs (11) erreicht; und
Befestigen des Zangenkopfes der genannten weiteren Einwegbiopsiezange am distalen Ende (14) des Einwegschlauchs (11) in der Arbeitsposition.

14. Verfahren nach Anspruch 13,
wobei das Einwegsystem eine Einführröhre aufweist;
wobei während des Schritts des Einführens der genannten weiteren Einwegbiopsiezange in den Arbeitskanal (22) des Einwegschlauchs (11) die Einführröhre die Bowdenzugseele der genannten weiteren Einwegbiopsiezange umgibt; und
wobei nach dem Schritt des Einführens der genannten weiteren Einwegbiopsiezange in den Arbeitskanal (22) des Einwegschlauchs (11) die Einführröhre aus dem Arbeitskanal (22) nach proximal entfernt wird.

## Claims

1. A disposable endoscopy and biopsy system (10) comprising:
a disposable tube (11) which surrounds at least one working passage (22) and which has endoscope fastening means (26, 28) for fastening the disposable tube (11) to an endoscope; and
at least one pair of disposable biopsy forceps (31) which can be introduced or is introduced into the working passage (22), wherein the pair of disposable biopsy forceps (31) has a forceps head (37) comprising at least two movable jaws (35) and a Bowden cable core (39) for actuating the jaws (35); and
wherein a fastening device (43) is provided for temporarily or permanently fastening the forceps head (37) to a distal end (14) of the disposable tube (11) in a working position in which the jaws (35) of the forceps head (37) project beyond the distal end (14) of the disposable tube (11),
**characterized in that**
the fastening device (43) is provided at the forceps head (37) and/or at the distal end (14) of the disposable tube (11).

2. A disposable system in accordance with claim 1,
wherein the fastening device (43) is adapted to fasten the forceps head (37) to the distal end (14) of the disposable tube (11) in an axially fixed, but rotationally movable manner in the working position.

3. A disposable system in accordance with one of the preceding claims,
wherein the fastening device (43) is adapted to establish a latching engagement between the distal end (14) of the disposable tube (11) and the forceps head (37) in said working position, and wherein at least one latch projection (47) is provided at the distal end (14) of the disposable tube (11) and at least one associated latch recess (45) is provided at the forceps head (37) or vice versa.

4. A disposable system in accordance with claim 3,
wherein the at least one latch projection (47) is an integral section of the disposable tube (11) or of the forceps head (37) which is preloaded in the direction of an engagement position due to inherent elasticity; or
wherein the at least one latch projection (47) is a separate element which is movably supported at the disposable tube (11) or at the forceps head (37) and which is preloaded in the direction of an engagement position by means of a preloading device.

5. A disposable system in accordance with one of the claims 1 or 2,
wherein the fastening device (43) is adapted to establish a magnetic coupling between the distal end of the disposable tube (11) and the forceps head (37) in said working position, and wherein at least one permanent magnet (54) is provided at the distal end (14) of the disposable tube (11) and at least one associated magnetic counter-element (54') is provided at the forceps head (37) or vice versa.

6. A disposable system in accordance with any one of the preceding claims,
wherein the fastening device (43) is adapted to release the forceps head (37) from the distal end (14) of the disposable tube (11) starting from said working position when the forceps head (37) is acted on by a predetermined force;
or
wherein the fastening device (43) has a materially continuous, shape-matched and/or force-transmitting combination means for permanently fastening the forceps head (37) to the distal end (14) of the disposable tube (11).

7. A disposable system in accordance with any one of the preceding claims,
wherein the disposable system furthermore has an introduction tube (41) which is adapted to be introduced into the working passage (22) together with the pair of disposable biopsy forceps (31), starting from a proximal end (16) of the disposable tube (11), in order to move the forceps head (37) into the working position, with the introduction tube (41) surrounding the Bowden cable core (39) in the introduced state, and with the introduction tube (41) being adapted to be removed to proximal from the working passage (22) while the forceps head (37) is in the working position; and
wherein the fastening device (43) is preferably adapted to fasten the forceps head (37) to the distal end (14) of the disposable tube (11) in an axially fixed, but rotationally movable manner in the working position, with the introduction tube (41) having an engagement means at a distal end which cooperates with an associated counter-engagement means at the forceps head (37) such that a rotational movement can be transmitted from the introduction tube (41) to the forceps head (37) which is fastened in the working position.

8. A disposable system in accordance with any one of the preceding claims,
wherein the disposable system furthermore has an actuation device (51) for displacing the Bowden cable core (39) relative to the disposable tube (11), with the actuation device (51) having at least one housing section (57) which is fastenable to a coupling device (53) at the proximal end (16) of the disposable tube (11), and with the actuation device (51) furthermore having at least one drive section (55) which is movably supported relative to the housing section (57) and which is fastenable to a proximal end of the Bowden cable core (39);
and/or
wherein the Bowden cable core (39) of the pair of disposable biopsy forceps (31) has a core composed of metal and a jacket composed of plastic.

9. A disposable system in accordance with any one of the preceding claims,
wherein the endoscope fastening means have a fixing device (26) by which the disposable tube (11) is rotationally fixedly and axially fixedly fixable to the distal end (14) of the endoscope, and wherein the endoscope fastening means furthermore have at least one guide device (28) by which the disposable tube (11) is displaceably fastenable to a respective section of the endoscope spaced apart from the distal end (14) of the endoscope;
and/or
wherein the disposable tube (11) additionally surrounds at least one flushing passage (24), with the disposable tube (11) having an outlet opening (20) of the flushing passage (24) at its distal end; and/or
wherein the disposable tube (11) has a cover (18) at its distal end (14) which seals the working passage (22) at the distal end (14) of the disposable tube (11) and which is configured to be opened by the application of force by means of the pair of disposable biopsy forceps (31) introduced into the working passage (22).

10. A method of configuring a disposable system in accordance with any one of the preceding claims, comprising the steps:
introducing the pair of disposable biopsy forceps (31) into the working passage (22) of the disposable tube (11) from its proximal end (16) until the forceps head (37) reaches the distal end (14) of the disposable tube (11); and
fastening the forceps head (37) to the distal end (14) of the disposable tube (11) in the working position.

11. A method in accordance with claim 10,
wherein the disposable system has an introduction tube (41); wherein the introduction tube (41) surrounds the Bowden cable core (39) of the pair of disposable biopsy forceps (31) during the step of introducing the pair of disposable biopsy forceps (31) into the working passage (22) of the disposable tube (11); and
wherein the introduction tube (41) is removed to proximal from the working passage (22) after the step of introducing the pair of disposable biopsy forceps (31) into the working passage (22) of the disposable tube (11).

12. A method in accordance with claim 10 or claim 11,
wherein the disposable system has an actuation device (51) for displacing the Bowden cable core (39) relative to the disposable tube (11), with the actuation device (51) having at least one housing section (57) and at least one drive section (55) which is movably supported relative to the housing section (57),
said method comprising the additional steps which are carried out after the step of fastening the forceps head (37) in the working position:
fastening the housing section (57) of the actuation device (51) to the proximal end (16) of the disposable tube (11); and
fastening the drive section (55) of the actuation device (51) to the proximal end of the Bowden cable core (39).

13. A method in accordance with any one of the claims 10 to 12, wherein the disposable system comprises at least one further pair of disposable biopsy forceps which has a forceps head comprising at least two movable jaws and a Bowden cable core for actuating the jaws,
said method comprising the additional steps:
releasing the forceps head (37) of said one pair of disposable biopsy forceps (31) from the distal end (14) of the disposable tube (11) and pulling said one pair of disposable biopsy forceps (31) out of the working passage (22) of the disposable tube (11);
introducing said further pair of disposable biopsy forceps into the working passage (22) of the disposable tube (11) from its proximal end (16) until the forceps head of said further pair of disposable biopsy forceps reaches the distal end (14) of the disposable tube (11); and
fastening the forceps head of said further pair of disposable biopsy forceps to the distal end (14) of the disposable tube (11) in the working position.

14. A method in accordance with claim 13,
wherein the disposable system has an introduction tube;
wherein the introduction tube surrounds the Bowden cable core of said further pair of disposable biopsy forceps during the step of introducing said further pair of disposable biopsy forceps into the working passage (22) of the disposable tube (11); and
wherein the introduction tube is removed to proximal from the working passage (22) after the step of introducing said further pair of disposable biopsy forceps into the working passage (22) of the disposable tube (11).

## Revendications

1. Système d'endoscopie et de biopsie (10) à usage unique, comportant :
un tuyau (11) à usage unique qui entoure au moins un canal de travail (22) et qui comprend des moyens de fixation d'endoscope (26, 28) pour fixer le tuyau (11) à usage unique sur un endoscope ; et
au moins une pince de biopsie (31) à usage unique qui est introduite ou susceptible d'être introduite dans le canal de travail (22), la pince de biopsie (31) à usage unique comprenant une tête de pince (37) pourvue d'au moins deux mâchoires mobiles (35) et une âme de câble Bowden (39) pour actionner les mâchoires (35) ;
dans lequel
il est prévu un dispositif de fixation (43) pour la fixation temporaire ou permanente de la tête de pince (37) à l'extrémité distale (14) du tuyau (11) à usage unique dans une position de travail dans laquelle les mâchoires (35) de la tête de pince (37) font saillie au-delà de l'extrémité distale (14) du tuyau (11) à usage unique,
**caractérisé en ce que**
le dispositif de fixation (43) est prévu sur la tête de pince (37) et/ou à l'extrémité distale (14) du tuyau (11) à usage unique.

2. Système à usage unique selon la revendication 1,
dans lequel
le dispositif de fixation (43) est adapté à fixer la tête de pince (37) de façon axialement fixe, mais mobile en rotation, à l'extrémité distale (14) du tuyau (11) à usage unique, dans la position de travail.

3. Système à usage unique selon l'une des revendications précédentes,
dans lequel
le dispositif de fixation (43) est adapté à établir une coopération par enclenchement entre l'extrémité distale (14) du tuyau (11) à usage unique et la tête de pince (37), dans ladite position de travail, au moins une saillie d'enclenchement (47) étant prévue à l'extrémité distale (14) du tuyau (11) à usage unique et au moins une cavité d'enclenchement (45) associée étant prévue sur la tête de pince (37), ou inversement.

4. Système à usage unique selon la revendication 3,
dans lequel
ladite au moins une saillie d'enclenchement (47) est une portion intégrale du tuyau (11) à usage unique ou de la tête de pince (37), qui est précontrainte en direction d'une position d'engagement grâce à l'élasticité propre ; ou
ladite au moins une saillie d'enclenchement (47) est un élément séparé qui est monté mobile sur le tuyau (11) à usage unique ou sur la tête de pince (37) et qui est précontraint en direction d'une position d'engagement à l'aide d'un moyen de précontrainte.

5. Système à usage unique selon l'une des revendications 1 ou 2,
dans lequel
le dispositif de fixation (43) est adapté à établir un couplage magnétique entre l'extrémité distale du tuyau (11) à usage unique et la tête de pince (37), dans ladite position de travail, au moins un aimant permanent (54) étant prévu à l'extrémité distale (14) du tuyau (11) à usage unique et au moins un élément magnétique complémentaire (54') associé étant prévu sur la tête de pince (37), ou inversement.

6. Système à usage unique selon l'une des revendications précédentes,
dans lequel
le dispositif de fixation (43) est adapté à libérer la tête de pince (37) vis-à-vis de l'extrémité distale (14) du tuyau (11) à usage unique à partir de ladite position de travail, lorsque la tête de pince (37) est sollicitée par une force prédéterminée ;
ou
le dispositif de fixation (43) comprend un moyen composite en coopération de matière, de forme et/ou de force pour la fixation permanente de la tête de pince (37) à l'extrémité distale (14) du tuyau (11) à usage unique.

7. Système à usage unique selon l'une des revendications précédentes,
dans lequel
le système à usage unique comprend en outre un tube d'introduction (41) qui est adapté à être introduit dans le canal de travail (22) conjointement avec la pince de biopsie (31) à usage unique à partir de l'extrémité proximale (16) du tuyau (11) à usage unique, pour amener la tête de pince (37) dans la position de travail, le tube d'introduction (41) entourant l'âme de câble Bowden (39), dans l'état introduit, et le tube d'introduction (41) étant adapté à être enlevé hors du canal de travail (22) en direction proximale, alors que la tête de pince (37) se trouve dans la position de travail ;
dans lequel
le dispositif de fixation (43) est de préférence adapté à fixer la tête de pince (37) de façon axialement fixe, mais mobile en rotation, à l'extrémité distale (14) du tuyau (11) à usage unique, dans la position de travail,
à une extrémité distale, le tube d'introduction (41) comprend un moyen d'engagement qui coopère avec un moyen d'engagement complémentaire associé sur la tête de pince (37) de telle sorte qu'un mouvement de rotation du tube d'introduction (41) est transmissible à la tête de pince (37) fixée dans la position de travail.

8. Système à usage unique selon l'une des revendications précédentes,
dans lequel
le système à usage unique comprend en outre un dispositif d'actionnement (51) pour décaler l'âme de câble Bowden (39) par rapport au tuyau (11) à usage unique,
le dispositif d'actionnement (51) comprend au moins une portion de boîtier (57) qui est susceptible d'être fixée sur un dispositif de couplage (53) à l'extrémité proximale (16) du tuyau (11) à usage unique, et
le dispositif d'actionnement (51) comprend en outre au moins une portion d'entraînement (55) qui est montée mobile par rapport à la portion de boîtier (57) et qui est susceptible d'être fixée à l'extrémité proximale de l'âme de câble Bowden (39) ;
et/ou
l'âme de câble Bowden (39) de la pince de biopsie (31) à usage unique comprend une âme en métal et une gaine en matière plastique.

9. Système à usage unique selon l'une des revendications précédentes,
dans lequel
les moyens de fixation d'endoscope présentent un dispositif d'attache (26) permettant d'attacher solidairement en rotation et de façon axialement fixe le tuyau (11) à usage unique à l'extrémité distale (14) de l'endoscope, et les moyens de fixation d'endoscope présentent en outre au moins un dispositif de guidage (28) permettant de fixer le tuyau (11) à usage unique de façon mobile en translation sur une portion respective de l'endoscope espacée de l'extrémité distale (14) de l'endoscope ;
et/ou
le tuyau (11) à usage unique entoure en supplément au moins un canal de rinçage (24), le tuyau (11) à usage unique présentant, à son extrémité distale (14), une ouverture de sortie (20) du canal de rinçage (24) ; et/ou
à son extrémité distale (14), le tuyau (11) à usage unique comprend un recouvrement (18) qui étanche le canal de travail (22) à l'extrémité distale (14) du tuyau (11) à usage unique et qui est réalisé pour être ouvert par l'action d'une force à l'aide de la pince de biopsie (31) à usage unique introduite dans le canal de travail (22).

10. Procédé pour configurer un système à usage unique selon l'une des revendications précédentes, comprenant les étapes consistant à :
introduire la pince de biopsie (31) à usage unique dans le canal de travail (22) du tuyau (11) à usage unique à partir de son extrémité proximale (16) jusqu'à ce que la tête de pince (37) atteigne l'extrémité distale (14) du tuyau (11) à usage unique ; et
fixer la tête de pince (37) à l'extrémité distale (14) du tuyau (11) à usage unique dans la position de travail.

11. Procédé selon la revendication 10,
dans lequel
le système à usage unique comprend un tube d'introduction (41) ; pendant l'étape d'introduction de la pince de biopsie (31) à usage unique dans le canal de travail (22) du tuyau (11) à usage unique, le tube d'introduction (41) entoure l'âme de câble Bowden (39) de la pince de biopsie (31) à usage unique ; et
après l'étape d'introduction de la pince de biopsie (31) à usage unique dans le canal de travail (22) du tuyau (11) à usage unique, le tube d'introduction (41) est enlevé hors du canal de travail (22) en direction proximale.

12. Procédé selon la revendication 10 ou 11,
dans lequel
le système à usage unique comprend un dispositif d'actionnement (51) pour décaler l'âme de câble Bowden (39) par rapport au tuyau (11) à usage unique, le dispositif d'actionnement (51) comprenant au moins une portion de boîtier (57) et au moins une portion d'entraînement (55) qui est montée mobile par rapport à la portion de boîtier (57) ;
comprenant les étapes supplémentaires mises en oeuvre après l'étape de la fixation de la tête de pince (37) dans la position de travail :
fixer la portion de boîtier (57) du dispositif d'actionnement (51) à l'extrémité proximale (16) du tuyau (11) à usage unique ; et
fixer la portion d'entraînement (55) du dispositif d'actionnement (51) à l'extrémité proximale de l'âme de câble Bowden (39).

13. Procédé selon l'une des revendications 10 à 12,
dans lequel
le système à usage unique comprend au moins une autre pince de biopsie à usage unique qui comprend une tête de pince pourvue d'au moins deux mâchoires mobiles et une âme de câble Bowden pour actionner les mâchoires ;
comprenant les étapes supplémentaires consistant à :
détacher la tête de pince (37) de ladite pince de biopsie (31) à usage unique vis-à-vis de l'extrémité distale (14) du tuyau (11) à usage unique et extraire ladite pince de biopsie (31) à usage unique hors du canal de travail (22) du tuyau (11) à usage unique ;
introduire ladite autre pince de biopsie à usage unique dans le canal de travail (22) du tuyau (11) à usage unique à partir de son extrémité proximale (16) jusqu'à ce que la tête de pince de ladite autre pince de biopsie à usage unique atteigne l'extrémité distale (14) du tuyau (11) à usage unique ; et
fixer la tête de pince de ladite autre pince de biopsie à usage unique à l'extrémité distale (14) du tuyau (11) à usage unique dans la position de travail.

14. Procédé selon la revendication 13,
dans lequel
le système à usage unique comprend un tube d'introduction ;
pendant l'étape d'introduction de ladite autre pince de biopsie à usage unique dans le canal de travail (22) du tuyau (11) à usage unique, le tube d'introduction entoure l'âme de câble Bowden de ladite autre pince de biopsie à usage unique ; et
après l'étape d'introduction de ladite autre pince de biopsie à usage unique dans le canal de travail (22) du tuyau (11) à usage unique, le tube d'introduction est enlevé hors du canal de travail (22) en direction proximale.
